# EUROPÄISCHE PATENTSCHRIFT

(11) **EP 2 237 034 B1**
(45) Veröffentlichungstag und Bekanntmachung des Hinweises auf die Patenterteilung: **24.07.2013**
(21) Anmeldenummer: 09157268.5
(22) Anmeldetag: 03.04.2009
(51) Int. Cl.: G01N 33/497, B60K 28/06

(54) **Vorrichtung und Verfahren zur Erkennung einer korrekten Anwendung eines Alkoholmessgerätes**
Device and method for recognising the correct usage of an alcohol measuring device
Dispositif et procédé de reconnaissance d'une application correcte d'un appareil de mesure d'alcoolémie

(43) Veröffentlichungstag der Anmeldung: 06.10.2010
(73) Patentinhaber: Dräger Safety AG & Co. KGaA, 23560 Lübeck (DE)
(72) Erfinder: Morley, Stefan, 23556, Lübeck (DE); Huth, Andreas, 23558, Lübeck (DE); Köhler, Ulrich, 23843, Bad Oldesloe (DE)
(74) Vertreter: UEXKÜLL & STOLBERG

(56) Entgegenhaltungen:
- EP-A- 0 172 169
- EP-A- 1 591 296
- US-A1- 2006 274 936
- US-A1- 2008 069 403
- US-A1- 2008 170 762

## Beschreibung

Die Erfindung betrifft eine Vorrichtung und ein Verfahren zur Erkennung einer korrekten Anwendung eines Alkoholmessgerätes von einer Testperson gemäß den Ansprüchen 1 und 11.

Alkoholmessgeräte werden insbesondere in der Kombination mit einer Wegfahrsperre eines Fahrzeuges, einem sogenannten "Interlock-System", angewendet. Ein Interlock-System besteht im Wesentlich aus zwei Komponenten: dem Alkoholmessgerät, das sich im Innenraum des Fahrzeuges befindet sowie einem Steuergerät, das in der Regel unter dem Armaturenbrett installiert ist und die Stromzufuhr zu einem Anlasserrelais des Fahrzeuges frei schaltet bzw. blockiert. Nach einem Einschalten der Zündung des Fahrzeuges fordert das Interlock-System den Bediener zur Abgabe einer Atemprobe auf. Das Messergebnis der Alkoholkonzentration entscheidet darüber, ob der Anlasser des Fahrzeuges frei geschaltet wird und damit der Motor gestartet werden kann. Grundsätzlich besteht bei der Anwendung des Interlock-Systems die Möglichkeit der Manipulation aufgrund einer Alkoholmessung durch eine zweite Person, die nicht der Fahrer ist. Zum Ausschluss einer solchen Umgehungsmöglichkeit weist in der Regel ein Interlock-System eine Wiederholungsfunktion der Alkoholmessung auf. Eine derartige Wiederholungsmessung wird beispielsweise nach einem nach dem Zufallsprinzip generierten Zeitintervall vom Fahrer verlangt. Dennoch besteht die Möglichkeit einer mehrmaligen Alkoholmessung von einer zweiten nicht das Fahrzeug steuernden Person.

Aus der EP 1 591 296 A1 ist ein Interlock-System bekannt, welches eine Videokamera umfasst, die selektiv Bilder speichert mit einem Lagesensor, der sicherstellt, dass die Videokamera korrekt den Tester erfasst. Das Videobild wird gespeichert und kann zur Identifikation an eine Auswertezentrale gesendet werden. Nachteilig bei dieser Vorrichtung ist jedoch, dass eine Identifikation des Testers zeitverzögert erfolgt bzw. ein Vergleich mit einem Bild des Testers hohe Anforderungen an eine Bildverarbeitung stellt.

Die US 2008/170 762 A1 offenbart ein Alkoholtestsystem umfassend wenigstens ein Atemalkoholmessgerät und eine Kamera. Hierbei wird von der Kamera, zur Feststellung der Identität des Fahrers, das Gesicht der Testperson fotografiert und mit einem vorherig registrierten Bild dieser Testperson verglichen.

Ausgehend vom Stand der Technik besteht die Aufgabe der vorliegenden Erfindung darin, eine Vorrichtung, die die Inbetriebnahme eines Fahrzeuges, einer Maschine oder einer Zutrittsbarriere durch eine alkoholisierte Bedienungsperson verhindert, derart zu verbessern, dass die Manipulationssicherheit erhöht wird.

Diese Aufgabe wird durch eine Vorrichtung und ein Verfahren zur Erkennung einer korrekten Anwendung eines Alkoholmessgerätes von einer Testperson mit den Merkmalen der unabhängigen Ansprüche gelöst.

In den davon abhängigen Ansprüchen sind vorteilhafte Ausgestaltungen und Weiterbildungen der Erfindung angegeben.

Die erfindungsgemäße Vorrichtung umfasst dabei wenigstens ein Alkoholmessgerät mit einem Signalgeber, eine Auswerte- und Steuereinheit und eine Kameraeinheit, wobei das Bildfeld der Kameraeinheit in einen inneren und einen äußeren Bildfeldbereich unterteilt ist, wobei der innere Bildfeldbereich wenigstens den Gesichtsbereich der Testperson erfasst, wobei das Alkoholmessgerät mit einem Signalgeber ausgeführt ist und die Kameraeinheit eingerichtet ist, ein von dem Signalgeber im inneren Bildfeldbereich abgegebenes Signal zu erfassen und die Auswerte- und Steuereinheit eingerichtet ist, dieses abgegebene Signal auszuwerten.

Mit der erfindungsgemäßen Vorrichtung wird aufgrund der Erfassung und Auswertung des Signals erkannt, ob das Alkoholmessgerät sich im inneren Bildfeldbereich der Kameraeinheit befindet. Die Kameraeinheit ist dabei so eingerichtet, dass der innere Bildfeldbereich der Kameraeinheit wenigstens den Gesichtsbereich der Testperson erfasst. Damit kann sichergestellt werden, dass das Alkoholmessgerät sich bei der Alkoholmessung im Bereich des Ortes der Probennahme befindet.

Die Kameraeinheit ist in vorteilhafter Weise als Infrarotkamera ausgeführt, wobei die Infrarotkamera vorzugsweise in einem Spektralbereich von 450 - 1050 Nanometern arbeitet. Die Empfindlichkeit der Kamera liegt bei einer Quanteneffizienz von mehr als 10 %. Dadurch wird sowohl eine Anwendung bei Tageslicht als auch bei aktiver nicht sichtbarer Beleuchtung im infraroten Spektralbereich ermöglicht. Der an dem Alkoholmessgerät vorgesehene Signalgeber ist ausgebildet, ein infrarotes Signal auszugeben, welches vorzugsweise alternierend blinkt. Durch die Anwendung einer Infrarotkamera und ein von der Infrarotkamera zu detektierendes Infrarotlichtsignal kann auch unter schlechten Lichtverhältnissen und bei Dunkelheit die Funktionalität der erfindungsgemäßen Vorrichtung gewährleistet werden.

Alternativ hierzu kann eine Kameraeinheit für den sichtbaren Spektralbereich verwendet werden, die ein optisches Lichtsignal des Signalgebers im inneren Bildfeldbereich erfassen kann, wobei das optische Lichtsignal vorzugsweise alternierend rot und gelb blinkt.

In einer weiteren vorteilhaften Ausbildung der erfindungsgemäßen Vorrichtung kann der Signalgeber als ein Licht reflektierendes Element ausgebildet sein, welches von einem Lichtsignal einer Lichtquelle beaufschlagt wird, wobei das reflektierende Licht im inneren Bildfeldbereich von der Kameraeinheit erfassbar ist.

In einer wiederum bevorzugten Ausführungsform ist das Alkoholmessgerät als Atemalkoholmessgerät ausgebildet, welches den Alkoholgehalt einer von der Testperson abgegebenen Atemprobe misst. Ferner kann das Alkoholmessgerät tragbar und an einem Körperteil des Benutzers vorzugsweise an den Armen befestigbar ausgebildetes Alkoholmessgerät sein, welches einen Alkoholgehalt über einen die Hautpermeation messenden Gassensor misst.

In einer weiteren vorteilhaften Ausbildung der erfindungsgemäßen Vorrichtung ist die Kameraeinheit eingerichtet, Bewegungen von dem inneren Bildfeldbereich zu dem äußeren Bildfeldbereich und/oder von dem äußeren Bildfeldbereich zu dem inneren Bildfeldbereich zu erfassen. Die Auswerte- und Steuereinheit ist ferner eingerichtet, diese Bewegungen auszuwerten. Der innere Bildfeldbereich und vorzugsweise der äußere Bildfeldbereich der Kameraeinheit kann dabei eine ovale Form aufweisen. Eine ovale Form wie beispielsweise die Form einer Ellipse ist geeignet, die Testperson in ihrer länglichen Achse von der Kameraeinheit zu erfassen. Ferner ermöglicht die ovale Form eine günstigere Skalierbarkeit.

Die erfindungsgemäße Vorrichtung ermöglicht das Gefährdungspotential einer unter dem Einfluss von Alkohol stehenden Bedienperson in einer sicherheitsrelevanten Einrichtung durch die Erhöhung der Manipulationssicherheit einer Alkoholmessung zu reduzieren.

Die erfindungsgemäße Vorrichtung kann Anwendung bei der Zutrittskontrolle sicherheitsrelevanter Einrichtungen, bei einem Interlock-System zum Starten eines Fahrzeuges, Schiffs oder Flugzeuges nach einer erfolgten Alkoholmessung bis hin zur Freigabe eines Bedienfeldes einer Maschine vorgesehen sein.

Die vorliegende Erfindung wird mit Bezug auf die beigefügten Zeichnungen detaillierter erläutert, wobei gleiche Bezugszeichen gleiche Figuren bezeichnen.

In der Zeichnung gilt:
- Figur 1: ist eine schematische Darstellung einer ersten Ausführung der erfindungsgemäßen Vorrichtung in einem Fahrzeug,
- Figur 2: ist eine schematische Darstellung einer zweiten Ausführung der erfindungsgemäßen Vorrichtung in einem Fahrzeug,
- Figur 3: ist eine schematische Darstellung einer dritten Ausführung der erfindungsgemäßen Vorrichtung in einem Fahrzeug,
- Figur 4: ist eine schematische Darstellung eines Atemalkoholmessgerätes mit einem Signalgeber und
- Figur 5: ist ein Blockschaltbild der erfindungsgemäßen Vorrichtung in einem Interlook-System.

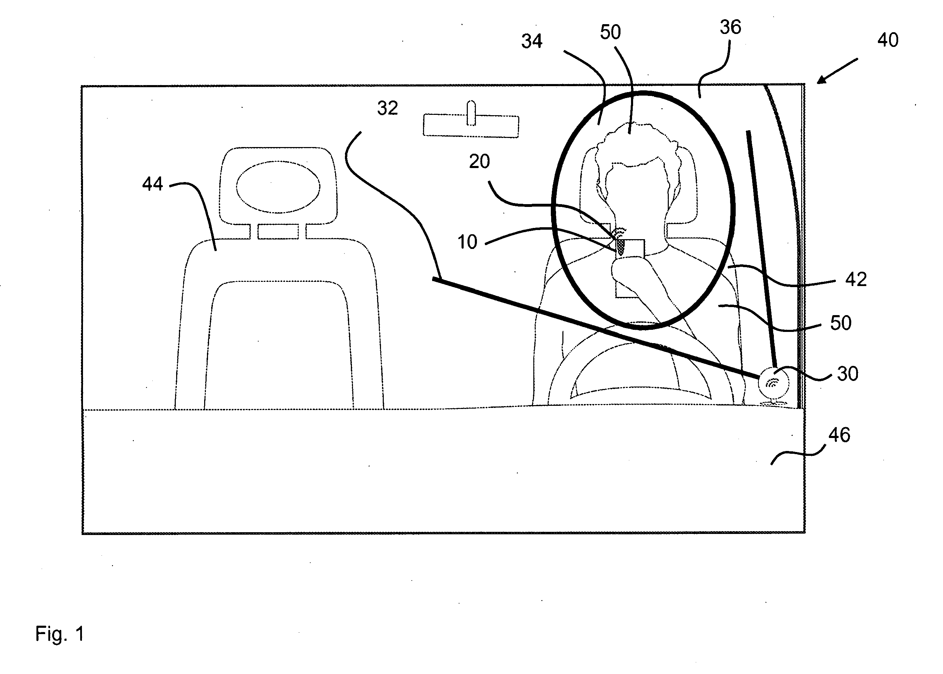

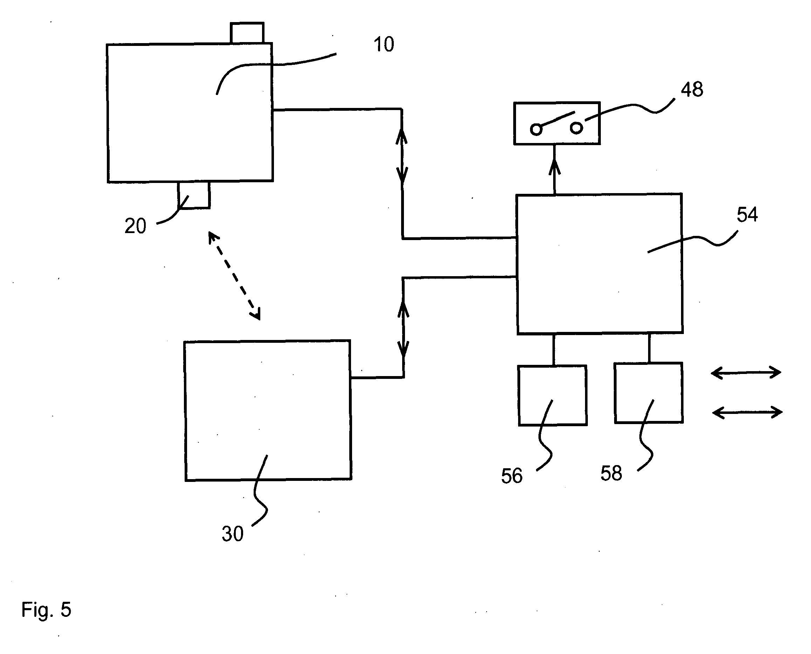

In Figur 1 ist die erfindungsgemäße Vorrichtung zur Erkennung einer korrekten Anwendung eines Alkoholmessgerätes 10 von einer Testperson 50 in Kombination mit einem Interlock-System in einem Fahrzeug 40 dargestellt. Die Figur 1 zeigt die vordere Ansicht des Fahrzeuges 40 mit einem Fahrersitz 42 und einem Beifahrersitz 44. Das Interlock-System besteht aus einem Alkoholmessgerät 10 und einem Auswerte- und Steuergerät 54 (Blockschaltbild Fig. 5), das sich unter einem Armaturenbrett 46 befindet. Das Auswerte- und Steuergerät 54 ist u.a. vorgesehen, die Stromzufuhr zu einem Anlasserrelais des Fahrzeuges 40 frei zuschalten bzw. zu blockieren. Das Alkoholmessgerät 10 ist als ein Atemalkoholmessgerät ausgebildet, welches den Alkoholgehalt einer von der Testperson 50 abgegebenen Atemprobe misst. Das Messergebnis der Atemalkoholkonzentration entscheidet darüber, ob eine Starteinrichtung 48 (Blockschaltbild Fig. 5) des Fahrzeuges 40 freigeschaltet wird und damit der Motor gestartet werden kann. Eine auf dem Armaturenbrett 46 angebrachte Kameraeinheit 30 weist ein Bildfeld 32 auf, welches in einen inneren Bildfeldbereich 34 und einen äußeren Bildfeldbereich 36 unterteilt ist. Die Kameraeinheit 30 ist auf dem Armaturenbrett 46 so angebracht, dass der innere Bildfeldbereich 34 wenigstens den Gesichtsbereich der Testperson 50 erfasst. Die Testperson 50 sitzt zur Alkoholmessung auf dem Fahrersitz 42. Die Kamera der Kameraeinheit 30 ist als Infrarotkamera ausgeführt. Der Spektralbereich der Infrarotkamera liegt in einem Bereich von 450 Nanometer bis 1050 Nanometer, bei einer Quanteneffizienz von mehr als 10 %. Dies ermöglicht sowohl den Einsatz der Kameraeinheit 30 bei Tageslicht als auch bei nicht sichtbarer Beleuchtung im infraroten Spektralbereich.

Das Alkoholmessgerät 10 weist ferner einen Signalgeber 20 auf, der während oder vor einer Alkoholmessung ein Signal ausgibt, wobei ein von dem Signalgeber 20 im inneren Bildfeldbereich 34 abgegebenes Signal von der Kameraeinheit 30 erfassbar ist. Eine Auswerte- und Steuereinheit 54 (dargestellt in Fig. 5) ist eingerichtet, dieses abgegebene Signal auszuwerten. Der Signalgeber 20 in der in Figur 1 dargestellten Ausführungsvariante gibt ein infrarotes Signal aus, das alternierend ein- und ausgeschaltet wird und von der als Infrarotkamera ausgebildeten Kameraeinheit 30 detektiert werden kann. Eine Signalmodulation des infraroten Signals durch ein alternierendes Ein- und Ausschalten erhöht die Zuverlässigkeit der erfindungsgemäßen Vorrichtung. Ein reproduziertes Modell eines Alkoholmessgerätes mit vorgesehenem Signalgeber würde somit von dem System erkannt werden. Ferner wird in diesem Schritt der Alkoholmessung mit der erfindungsgemäßen Vorrichtung sichergestellt, dass sich das Alkoholmessgerät bei der Alkoholmessung im Bereich des Ortes der Probennahme befindet, nämlich im Bereich des Fahrersitzes.

Ferner kann in einem vorherigen Schritt mit der erfindungsgemäßen Vorrichtung sichergestellt werden, dass sich keine weitere Person im Bereich des Fahrersitzes 42 befindet. Dazu wird von der Kameraeinheit 30 eine Anzahl von Personen im inneren Bildfeldbereich 34 ermittelt, wobei bei der Ermittlung von mehr als einer Person im inneren Bildfeldbereich 34 eine Signalgabe erfolgt. In vorteilhafter Weise wird von der Kameraeinheit 30 ein Bild des inneren Bildfeldbereiches erfasst und gespeichert.

Sowohl das Alkoholmessgerät 10 als auch die Kameraeinheit 30 sind mit der Auswerte- und Steuereinheit 54 verbunden. Nach einer Betätigung der Starteinrichtung 48 des Fahrzeuges 40 signalisiert das Interlock-System der Testperson 50 den Beginn einer Alkoholmessung. Die Kameraeinheit 30 wird aktiviert und überwacht in dem inneren Bildfeldbereich 34 ein Vorhandensein des infraroten Signals innerhalb des inneren Bildfeldbereiches 34. Wird kein infrarotes Signal von der Kameraeinheit 30 innerhalb des inneren Bildfeldbereiches 34 erkannt, kann von dem Interlock-System eine neue Alkoholmessung gefordert werden.

Alternativ ist es auch möglich, dass die Kameraeinheit 30 mit der Betätigung der Starteinrichtung 48 des Fahrzeuges 40 durch die Testperson 50 in Bereitschaft geht, aber erst nach einer Bestätigung an dem Alkoholmessgerät 10, beispielsweise durch Betätigung einer an dem Alkoholmessgerät 10 vorgesehenen Aktivierungstaste aktiviert wird.

In einer weiteren Ausgestaltung der erfindungsgemäßen Vorrichtung ist die Kameraeinheit eingerichtet, zu Beginn der Alkoholmessung alle Veränderungen innerhalb des inneren Bildfeldbereiches 34 und zumindest teilweise des äußeren Bildfeldbereiches 36 zu registrieren. In einer besonderen Ausgestaltung des erfindungsgemäßen Verfahrens werden Veränderungen in einem Grenzbereich 38, beispielsweise durch eine Bewegung der Testperson 50, aus dem inneren Bildfeldbereich 34 in den äußeren Bildfeldbereich 36 oder einer weiteren Person von dem äußeren Bildfeldbereich 36 in den inneren Bildfeldbereich 34 von der Kameraeinheit 30 erfasst und von der Auswerte- und Steuereinheit 54 ausgewertet. Die Auswerte- und Steuereinheit 54 signalisiert eine nicht korrekte Anwendung des Alkoholmessgerätes 10. Das bedeutet, dass während einer Alkoholmessung die Testperson 50 auf den Fahrersitz 42 des Fahrzeuges 40 (im inneren Bildfeldbereich 34 der Kameraeinheit 30) verweilen muss. Während der Alkoholmessung sendet der an dem Alkoholmessgerät 10 vorgesehene Signalgeber 20 ein Infrarotes Signal, welches von der Kameraeinheit 30 registrierbar ist. Die Alkoholmessung wird von der Auswerte- und Steuereinheit 54 als gültig erkannt, wenn während der Dauer der Alkoholmessung keine Veränderung im Grenzbereich des inneren und äußeren Bildfeldbereiches 34 und 36 von der Kameraeinheit 30 detektiert worden ist und die Kameraeinheit 30 das infrarote Signal innerhalb des inneren Bildfeldbereiches 34 registriert hat. Wird kein infrarotes Signal von der Kameraeinheit 30 innerhalb des inneren Bildfeldbereiches 34 erkannt und / oder ist eine Veränderung im Grenzbereich des inneren und äußeren Bildfeldbereiches 34 und 36 von der Auswerte- und Steuereinheit 54 erkannt worden, kann von dem Interlock-System eine neue Alkoholmessung gefordert werden. Bei einer als gültig erklärten Alkoholmessung wird bei Vorliegen des Alkoholmesswertes im erlaubten Bereich die Starteinrichtung 48 des Fahrzeugs 40 frei geschalten und damit kann der Motor gestartet werden. Alternativ ist es auch in einer abgestuften Handlungsfolge möglich, bei einem Alkoholmesswert im erlaubten Bereich trotz nicht detektierten Signals des Signalgebers 20 des Alkoholmessgerätes 10 im inneren Bildfeldbereich 34 ein Starten des Fahrzeugs 40 zu ermöglichen. In diesen Fällen wird wenigstens das Bild des inneren Bildfeldbereiches 34 der Kameraeinheit 30 erfasst und von der Auswerte- und Steuereinheit 54 gespeichert.

Bei dem erfindungsgemäßen Verfahren wird die nicht korrekte, regelwidrige Benutzung des Interlock-Systems dadurch dokumentiert, dass bei erkannter Regelwidrigkeit entweder bei einem nicht erkannten Alkoholmessgerät 10 im inneren Bildfeldbereich 34 oder einer Bewegung durch einen nicht erlaubten Grenzbereich 38 ebenfalls ein Triggersignal für die Dokumentation des Ereignisses mittels der Kameraeinheit 30 gesetzt wird.

In einem alternativen Verfahren der weiteren Verfahrensfolge, d.h. in dem eine Freigabe der Starteinrichtung 48 ausschließlich an den Messwert der Alkoholmessung geknüpft wird, wird die potenziell regelwidrige Benutzung des Fahrzeuges 40 von der Testperson 50 als Ereignis dokumentiert, das einen Hinweis auf diese potenzielle Regelverletzung beinhaltet. Eine Dokumentation des Ereignisses kann beispielsweise durch eine Textbeschreibung in der Auswerte- und Steuereinheit 54 erfolgen.

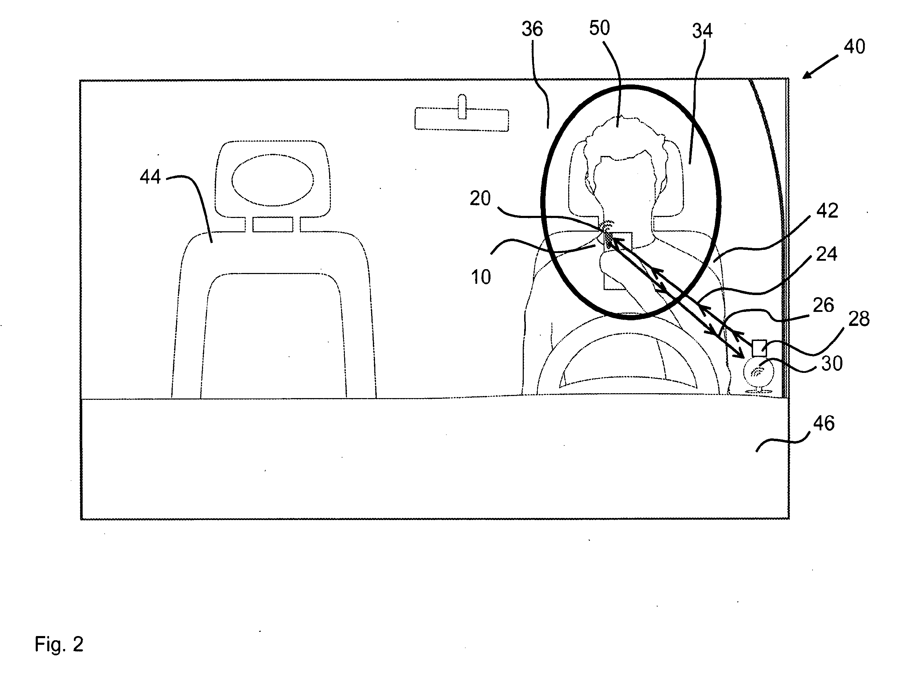

In Figur 2 ist die Kameraeinheit 30 als eine Kamera für den sichtbaren Spektralbereich ausgebildet. Die Kameraeinheit 30 ist mit einer integrierten Lichtquelle 28 ausgeführt. Die Kameraeinheit 30 ist auf einem Armaturenbrett 46 des Fahrzeuges 40 angebracht, wobei die Lichtquelle 28 derart ausgerichtet ist, einen als ein Licht reflektierendes Element ausgebildeten Signalgeber 20 anzustrahlen. Das von der Kameraeinheit 30 in den inneren Bildfeldbereich 34 ausgesendete Licht wird bei Anwesenheit des Alkoholmessgerätes 10 innerhalb des inneren Bildfeldbereiches 34 von dem Signalgeber 20 reflektiert. Das reflektierte Licht kann von der Kameraeinheit 30 detektiert werden. Alternativ kann die Lichtquelle 28 auch an einer anderen Stelle innerhalb des Fahrzeuges vorgesehen sein, beispielsweise direkt auf dem Armaturenbrett 46. Der Signalgeber 20 kann vorzugsweise als gleichseitiges Dreieck ausgebildet sein, wodurch durch Triangulation bei einer einmaligen Justierung beim Einbau der erfindungsgemäßen Vorrichtung in das Fahrzeug 40 die genaue Position des Alkoholmessgerätes ermittelt werden kann. Eine Signalmodulation des Lichtsignals der Lichtquelle 28 durch ein alternierendes Ein- und Ausschalten erhöht wiederum (wie bei der Ausführung der Figur 1) die Zuverlässigkeit der erfindungsgemäßen Vorrichtung. Alternativ dazu und insbesondere in Kombination mit einer Kameraeinheit 30 für den sichtbaren Spektralbereich kann der Signalgeber 20 des Alkoholmessgerätes 10 ausgebildet sein, ein optisches Signal auszugeben, welches alternierend rotes und gelbes Licht umfasst (nicht dargestellt).

Wie aus den Figuren 1 und 2 ersichtlich, kann der innere Bildfeldbereich 34 eine ovale Form aufweisen. Eine ovale Form in Form einer Ellipse gestattet eine gute Skalierbarkeit und eine optimale Erfassung der Testperson 50 in ihrer Längsachse.

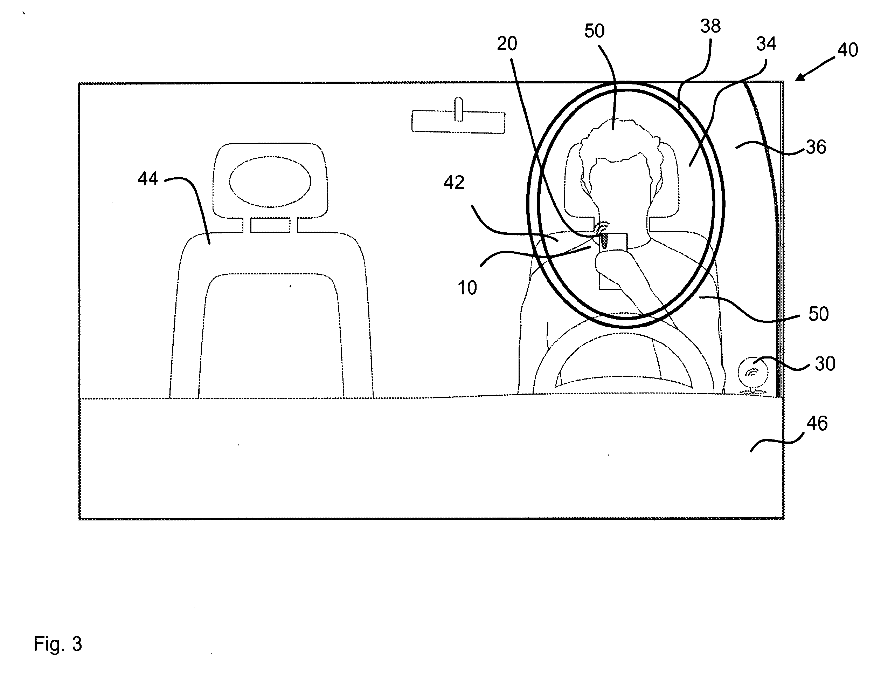

In der in Figur 3 dargestellten Ausführungsvariante sind der innere Bildfeldbereich 34 und der äußere Bildfeldbereich 36 nicht zusammenhängend, sondern weisen einen Grenzbereich 38 auf. Der Grenzbereich 38 ist vorzugsweise als regelmäßiger Streifen in ovaler Form um die Position des Fahrers, also der Testperson 50, herum ausgeführt. Der Grenzbereich 38 kann alternativ dazu auch unregelmäßig ausgebildet sein. Veränderungen innerhalb des Grenzbereiches 38 zwischen dem inneren Bildfeldbereich 34 und dem äußeren Bildfeldbereich 36 können von der Kameraeinheit 30 registriert werden. Eine Bewegung von dem äußeren Bildfeldbereich 36 durch den Grenzbereich 38 in den inneren Bildfeldbereich 34 hinein während der Alkoholmessung kann von der erfindungsgemäßen Vorrichtung erfasst und dokumentiert werden.

Alternativ zu den Ausführungen der Figur 1 bis 3 kann die Kameraeinheit 30 auch an anderer Position innerhalb der Fahrgastzelle des Fahrzeuges 40 vorgesehen sein, beispielsweise an einem Rückspiegel. In allen Fällen wird die Kameraeinheit 30 in dem Fahrzeug 40 so angebracht, dass sie den Bereich des Fahrersitzes 42 vollständig in ihrem Bildfeld 32 hat. Dies kann neben den erwähnten Positionen auch im Bereich der Mittelkonsole sein oder aber auch an der Frontscheibe in der Nähe der A-Säule des Fahrzeuges 40. Die Kameraeinheit 30 kann so fixiert werden, dass sie während des Zeitraums, in dem das Interlock-System in dem Fahrzeug 40 installiert ist, fest justiert ist, aber nach Ende des Installationszeitraums ohne sichtbare Spuren wieder entfernt werden kann. Vorzugsweise sollte die Kameraeinheit an einer Befestigungsstelle eine Siegelverklebung aufweisen.

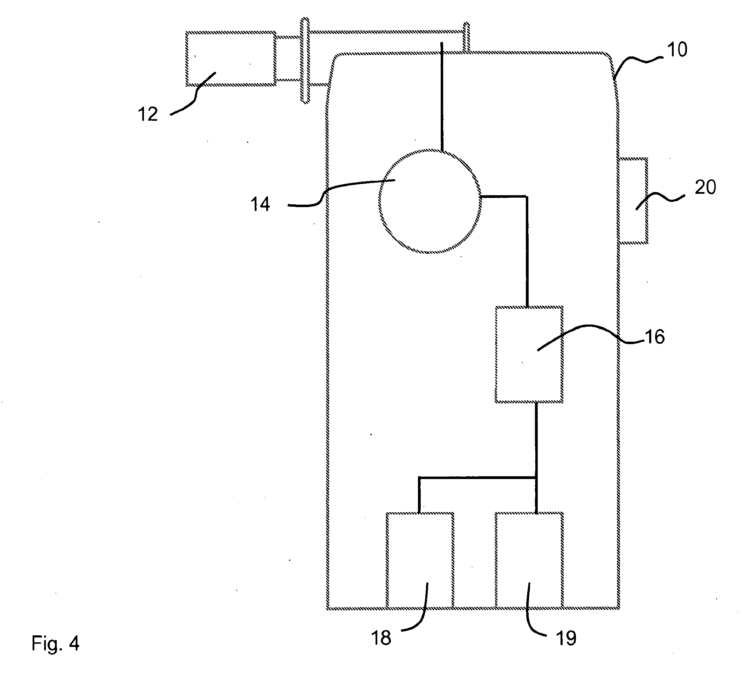

Die Figur 4 zeigt ein Alkoholmessgerät 10 in schematischer Ansicht. Das Alkoholmessgerät 10 ist als ein Atemalkoholmessgerät ausgeführt, welches die Atemalkoholkonzentration einer Testperson 50 misst. Dazu gibt die Testperson 50 durch ein Mundstück 12 eine Atemprobe in das Alkoholmessgerät 10, wobei die Atemalkoholkonzentration mit einem elektrochemischen Sensor 14 und einer anschließenden Messauswerteeinrichtung 16 ausgeführt ist. Der Signalgeber 20 ist an der gegenüberliegenden Seite des Mundstückes 12 vorgesehen. Ferner weist das Alkoholmessgerät 10 einen Kommunikationsanschluss für eine Kabelmontage 18 und / oder einen Kommunikationsanschluss für eine Funk getragene Kommunikation 19 zur Auswerte- und Steuereinheit 54 auf. Während der Benutzung des Fahrzeuges 40 durch die Testperson 50 werden alle relevanten Ereignisse, wie beispielsweise Datum, Uhrzeit, Abgabe oder Verweigerung einer Alkoholmessung, gemessene Alkoholkonzentrationen sowie eine nicht korrekte Anwendung des Alkoholmessgerätes 10 in einer Speichereinheit 56 aufgezeichnet (dargestellt in der Figur 5). Die Speichereinheit 56 ist vorzugsweise mit der Auswerte- und Steuereinheit 54 verbunden oder vorzugsweise in selbiger integriert.

In einer weiteren Ausführung kann das komplette Interlock-System mittels eines drahtlosen Kommunikationssystems mit einer zentralen Speichereinheit verbunden sein, auf welche die Daten in regelmäßigen Abständen übertragen werden. Kriterien für die Übertragung können sowohl zeitliche Abstände, beispielsweise Tage sein, oder das Speichervolumen der im Fahrzeug 40 integrierten Speichereinheit 56 oder eine Kombination aus beiden.

Die Anwendung der erfindungsgemäßen Vorrichtung in Kombination mit einem Interlock-System in einem Fahrzeug 40 stellt nur eine Ausführungsvariante dar. Alternativ hierzu kann die erfindungsgemäße Vorrichtung in einer Zugangskontrolle für industrielle Sicherheitsbereiche, beispielsweise in der chemischen Industrie, oder aber auch beispielsweise für die Freigabe eines Bedienfeldes einer Maschine integriert sein.

Während die vorliegende Erfindung unter Bezugnahme auf die bevorzugten Ausführungsbeispiele beschrieben worden ist, sind dem Fachmann verschiedene Änderungen und Modifikation klar. All diese Änderungen und Modifikationen sollen in den Schutzbereich der angefügten Ansprüche fallen.

### BEZUGSZEICHENLISTE

- 10: Alkoholmessgerät
- 12: Mundstück
- 14: Sensor
- 16: Messauswerteeinrichtung
- 18: Kommunikationsanschluss für Kabelmontage
- 19: Kommunikationsanschluss für funkgetragene Kommunikation zur Auswerte- und Steuereinheit
- 20: Signalgeber
- 22: Licht reflektierendes Element
- 24: Sendesignal
- 26: Reflektorsignal
- 28: Lichtquelle
- 30: Kameraeinheit
- 32: Bildfeld der Kameraeinheit
- 34: innerer Bildfeldbereich
- 36: äußerer Bildfeldbereich
- 38: Grenzbereich
- 40: Fahrzeug
- 42: Fahrersitz
- 44: Beifahrersitz
- 46: Armaturenbrett
- 48: Starteinrichtung
- 50: Testperson
- 52: Aufforderungseinheit
- 54: Auswerte- und Steuereinheit
- 56: Speichereinheit
- 58: Sende- und Empfangseinheit

## Patentansprüche

1. Vorrichtung zur Erkennung einer korrekten Anwendung eines Alkoholmessgerätes (10) von einer Testperson (50)
umfassend wenigstens das Alkoholmessgerät (10) eine Auswerte- und Steuereinheit (54) und eine Kameraeinheit (30),
wobei das Bildfeld (32) der Kameraeinheit (30) in einen inneren Bildfeldbereich (34) und einen äußeren Bildfeldbereich (36) unterteilt ist, **dadurch gekennzeichnet, dass**
das Alkoholmessgerät (10) mit einem Signalgeber (20) ausgeführt ist,
der innere Bildfeldbereich (34) wenigstens den Gesichtbereich der Testperson (50) erfasst und die Kameraeinheit (30) eingerichtet ist, ein von dem Signalgeber (20) im inneren Bildfeldbereich (34) abgegebenes Signal zu erfassen und die Auswerte- und Steuereinheit (54) eingerichtet ist, dieses abgegebene Signal auszuwerten.

2. Vorrichtung nach Anspruch 1, **dadurch gekennzeichnet, dass** das Alkoholmessgerät (10) den Alkoholgehalt einer von der Testperson (50) abgegebenen Atemprobe misst.

3. Vorrichtung nach einem der vorhergehenden Ansprüche, **dadurch gekennzeichnet, dass** die Kameraeinheit (30) eine Infrarot-Kamera aufweist und der Signalgeber (20) ein infrarotes Signal ausgibt, das vorzugsweise alternierend ein- und ausschaltbar ist.

4. Vorrichtung nach einem der vorhergehenden Ansprüche, **dadurch gekennzeichnet, dass** die Kameraeinheit (30) im sichtbaren Spektralbereich arbeitet.

5. Vorrichtung nach Anspruch 4, **dadurch gekennzeichnet, dass** der Signalgeber (20) ein optisches Signal ausgibt, welches vorzugsweise alternierend rotes und gelbes Licht umfasst oder der Signalgeber (20) als ein Licht reflektierendes Element (22) ausgebildet ist und eine Lichtquelle (28) zur Beleuchtung des Signalgebers (20) vorgesehen ist.

6. Vorrichtung nach einem der vorhergehenden Ansprüche, **dadurch gekennzeichnet, dass** der innere Bildfeldbereich (34) und vorzugsweise der äußere Bildfeldbereich (36) der Kameraeinheit (30) eine ovale Form aufweise.

7. Vorrichtung nach einem der vorhergehenden Ansprüche, **dadurch gekennzeichnet, dass** die Kameraeinheit (30) eingerichtet ist, Bewegungen von dem inneren Bildfeldbereich (34) zu dem äußeren Bildfeldbereich (36) und/oder von dem äußeren Bildfeldbereich (36) zu dem inneren Bildfeldbereich (34) zu erfassen und die Auswerte- und Steuereinheit (54) eingerichtet ist, diese Bewegungen auszuwerten.

8. Vorrichtung nach einem der vorhergehenden Ansprüche, **dadurch gekennzeichnet, dass** die Grenze zwischen dem inneren Bildfeldbereich (34) und dem äußeren Bildfeldbereich (36) als ein Grenzbereich (38) ausgebildet ist.

9. Vorrichtung nach einem der vorhergehenden Ansprüche, **dadurch gekennzeichnet, dass** eine Speichereinheit (56) zum Aufzeichnen von Bildern des inneren Bildfeldbereiches (34) vorgesehen ist, die vorzugsweise als ein Teil der Kameraeinheit (30) ausgebildet ist.

10. Vorrichtung zur Erkennung einer korrekten Anwendung eines Alkoholmessgerätes (10) von einer Testperson (50) nach einem der vorhergehenden Ansprüche in Kombination mit einem System zur Überprüfung des Alkoholgehaltes der Testperson (50), umfassend wenigstens:
ein Fahrzeug (40) und eine Wegfahrsperre,
wobei die Wegfahrsperre wenigstens mit dem Alkoholmessgerät (10) in Verbindung steht und
die Kameraeinheit (30) im Fahrzeug (40) so angebracht ist, dass der innere Bildfeldbereich (34) den Bereich eines Fahrersitzes (42) vollständig umfasst.

11. Verfahren zur Erkennung einer korrekten Anwendung eines Alkoholmessgerätes (10) von einer Testperson (50) mit den folgenden Schritten:
a) eine Kameraeinheit (30), deren Bildfeld (32) in einen inneren Bildfeldbereich (34) und einen äußeren Bildfeldbereich (36) unterteilt ist, registriert den inneren Bildfeldbereich (34) und den äußeren Bildfeldbereich (36) zu Beginn der Alkoholmessung,
b) eine an dem Alkoholmessgerät (10) vorgesehener Signalgeber (20) sendet ein Signal während der Alkoholmessung, welches von der Kameraeinheit (30) registrierbar ist,
c) eine Auswerte- und Steuereinheit (54) wertet das Signal aus und
d) die Alkoholmessung wird von einer Auswerteeinheit- und Steuereinheit (54) als gültig erkannt, wenn während der Alkoholmessung das Signal innerhalb des inneren Bildfeldbereiches (34) detektiert wurde.

12. Verfahren nach Anspruch 11, **dadurch gekennzeichnet, dass** die Kameraeinheit (30) vor der Alkoholmessung aktiviert wird, vorzugsweise nach einer Einleitung des Startvorgangs eines Fahrzeuges (40).

13. Verfahren nach Anspruch 11 oder 12, **dadurch gekennzeichnet, dass** im Schritt a) von der Kameraeinheit (30) eine Anzahl von Personen im inneren Bildfeldbereich (34) ermittelt wird, wobei vorzugsweise bei einer Ermittlung von mehr als einer Person im inneren Bildfeldbereich (34) eine Signalgabe erfolgt und / oder die Alkoholmessung nicht gestartet wird.

14. Verfahren nach Anspruch 11, **dadurch gekennzeichnet, dass** im Verfahrensschritt b) der Signalgeber (20) ein infrarotes Signal sendet, das vorzugsweise alternierend ein- und ausgeschaltet wird oder wenigstens ein Lichtsignal sendet, welches vorzugsweise alternierend rotes und gelbes Licht umfasst und / oder die Lichtstärke eines Lichtsignals variiert wird.

15. Verfahren nach Anspruch 11, **dadurch gekennzeichnet, dass** die Kameraeinheit (30) Veränderungen an der Grenze zwischen dem inneren Bildfeldbereich (34) und dem äußeren Bildfeldbereich (36) ab Beginn der Alkoholmessung erfasst und die Auswerte- und Steuereinheit (54) diese Veränderungen auswertet.

## Claims

1. Apparatus for recognizing a correct use of an alcohol measuring device (10) by a test person (50) comprising at least the alcohol measuring device (10), an evaluation and control unit (54) and a camera unit (30), wherein the image field (32) of the camera unit (30) is divided into an inner image field portion (34) and an outer image field portion (36), **characterized in that**
the alcohol measuring device (10) is realized with a signal generator (20), the inner image field portion (34) covers at least the face region of the test person (50), and the camera unit (30) is adapted to detect a signal provided by the signal generator (20) in the inner image field portion (34), and the evaluation and control unit (54) is adapted to evaluate this provided signal.

2. Apparatus according to claim 1, **characterized in that** the alcohol measuring device (10) measures the alcohol concentration of a breath sample provided by the test person (50).

3. Apparatus according to any of the preceding claims, **characterized in that** the camera unit (30) comprises an infrared camera and the signal generator (20) provides an infrared signal, which preferably can be switched on and off alternatingly.

4. Apparatus according to any of the preceding claims, **characterized in that** the camera unit (30) operates in the visible spectral range.

5. Apparatus according to claim 4, **characterized in that** the signal generator (20) provides an optical signal, which preferably comprises alternatingly red and yellow light, or the signal generator (20) is constructed as a light reflecting element (22) and a light source (28) is provided for illuminating the signal generator (20).

6. Apparatus according to any of the preceding claims, **characterized in that** the inner image field portion (34) and, preferably, the outer image field portion (36) of the camera unit (30) have an oval shape.

7. Apparatus according to any of the preceding claims, **characterized in that** the camera unit (30) is adapted to detect movements from the inner image field portion (34) to the outer image field portion (36) and/or from the outer image field portion (36) to the inner image field portion (34), and the evaluation and control unit (54) is adapted to evaluate these movements.

8. Apparatus according to any of the preceding claims, **characterized in that** the boundary between the inner image field portion (34) and the outer image field portion (36) is constructed as a boundary region (38).

9. Apparatus according to any of the preceding claims, **characterized in that** a storage unit (56) for recording images of the inner image field portion (34) is provided, which storage unit (56) is preferably constructed as a part of the camera unit (30).

10. Apparatus for recognizing a correct use of an alcohol measuring device (10) by a test person (50) according to any of the preceding claims in combination with a system for checking the alcohol concentration of the test person (50), comprising at least:
a vehicle (40) and an immobilizer,
wherein the immobilizer is connected at least with the alcohol measuring device (10) and
the camera unit (30) is installed in the vehicle (40) in such a manner that the inner image field portion (34) completely covers the region of a driver seat (42).

11. Method for recognizing a correct use of an alcohol measuring device (10) by a test person (50) comprising the following steps:
a) a camera unit (30), the image field (32) of which is divided into an inner image field portion (34) and an outer image field portion (36), registers the inner image field portion (34) and the outer image field portion (36) at the beginning of the alcohol measurement,
b) a signal generator (20) provided on the alcohol measuring device (10) transmits a signal during the alcohol measurement, which signal can be registered by the camera unit (30),
c) an evaluation and control unit (54) evaluates the signal, and
d) the alcohol measurement is recognized as valid by an evaluation unit and control unit (54), if during the alcohol measurement the signal was detected within the inner image field portion (34).

12. Method according to claim 11, **characterized in that** the camera unit (30) is activated prior to the alcohol measurement, preferably following an initiation of the start process of the vehicle (40).

13. Method according to claim 11 or 12, **characterized in that** in step a) a number of persons within the inner image field portion (34) is determined by the camera unit (30), wherein preferably upon a determination of more than one person within the inner image field portion (34) a signal generation is carried out and/or the alcohol measurement is not started.

14. Method according to claim 11, **characterized in that** in method step b) the signal generator (20) transmit an infrared signal, which is preferably switched on and off alternatingly, or transmits at least one light signal, which preferably comprises alternatingly red and yellow light, and/or the light intensity of a light signal is varied.

15. Method according to claim 11, **characterized in that** the camera unit (30) detects changes at the boundary between the inner image field portion (34) and the outer image field portion (36) starting with the beginning of the alcohol measurement, and the evaluation and control unit (54) evaluates these changes.

## Revendications

1. Dispositif de détection d'une utilisation correcte d'un éthylomètre (10) par une personne (50), comprenant au moins l'éthylomètre (10), une unité d'analyse et de commande (54) et une unité de prise de vues (30), le champ d'image (32) de l'unité de prise de vues (30) étant divisé en une zone de champ d'image intérieure (34) et une zone de champ d'image extérieure (36), **caractérisé en ce que** l'éthylomètre (10) est réalisé avec un émetteur de signaux (20), la zone de champ d'image intérieure (34) couvre au moins la zone du visage de la personne (50), l'unité de prise de vues (30) est conçue pour détecter un signal émis par l'émetteur de signaux (20) dans la zone de champ d'image intérieure (34) et l'unité d'analyse et de commande (54) est conçue pour analyser ce signal émis.

2. Dispositif selon la revendication 1, **caractérisé en ce que** l'éthylomètre (10) mesure la teneur en alcool d'un échantillon d'air expiré par la personne (50).

3. Dispositif selon une des revendications précédentes, **caractérisé en ce que** l'unité de prise de vues (30) comprend une caméra infrarouge, et l'émetteur de signaux (20) émet un signal infrarouge qui est de préférence alternativement activable et désactivable.

4. Dispositif selon une des revendications précédentes, **caractérisé en ce que** l'unité de prise de vues (30) fonctionne dans le domaine spectral visible.

5. Dispositif selon la revendication 4, **caractérisé en ce que** l'émetteur de signaux (20) émet un signal optique qui comprend de préférence alternativement une lumière rouge et une lumière jaune, ou l'émetteur de signaux (20) est réalisé sous la forme d'un élément (20) qui réfléchit la lumière et une source de lumière (28) destinée à éclairer l'émetteur de signaux (20) est prévue.

6. Dispositif selon une des revendications précédentes, **caractérisé en ce que** la zone de champ d'image intérieure (34) et de préférence la zone de champ d'image extérieure (36) de l'unité de prise de vues (30) présentent une forme ovale.

7. Dispositif selon une des revendications précédentes, **caractérisé en ce que** l'unité de prise de vues (30) est conçue pour enregistrer des mouvements de la zone de champ d'image intérieure (34) vers la zone de champ d'image extérieure (36) et/ou de la zone de champ d'image extérieure (36) vers la zone de champ d'image intérieure (34), et l'unité d'analyse et de commande (54) est conçue pour analyser ces mouvements.

8. Dispositif selon une des revendications précédentes, **caractérisé en ce que** la limite entre la zone de champ d'image intérieure (34) et la zone de champ d'image extérieure (36) est réalisée sous la forme d'une zone limite (38).

9. Dispositif selon une des revendications précédentes, **caractérisé en ce qu'**il est prévu, pour enregistrer des images de la zone de champ d'image intérieure (34), une unité de mémorisation (56) qui est de préférence réalisée comme une partie de l'unité de prise de vues (30).

10. Dispositif de détection d'une utilisation correcte d'un éthylomètre (10) par une personne (50) selon une des revendications précédentes en combinaison avec un système de vérification de l'alcoolémie de la personne (50), comprenant au moins un véhicule (40) et un système antidémarrage, le système antidémarrage étant relié au moins à l'éthylomètre (10) et l'unité de prise de vues (30) étant disposée dans le véhicule (40) de façon que la zone de champ d'image intérieure (34) couvre entièrement la zone d'un siège de conducteur (42).

11. Procédé de détection d'une utilisation correcte d'un éthylomètre (10) par une personne (50), comportant les étapes suivantes :
a) une unité de prise de vues (30), dont le champ d'image (32) est divisé en une zone de champ d'image intérieure (34) et une zone de champ d'image extérieure (36), enregistre la zone de champ d'image intérieure (34) et la zone de champ d'image extérieure (36) au début de la mesure d'alcool,
b) un émetteur de signaux (20) prévu sur l'éthylomètre (10) envoie, pendant la mesure d'alcool, un signal qui peut être enregistré par l'unité de prise de vues (30),
c) une unité d'analyse et de commande (54) analyse le signal et
d) la mesure d'alcool est reconnue comme valable par une unité d'analyse et de commande (54) si le signal a été détecté à l'intérieur de la zone de champ d'image intérieure (34) pendant la mesure d'alcool.

12. Procédé selon la revendication 11, **caractérisé en ce que** l'unité de prise de vues (30) est activée avant la mesure d'alcool, de préférence après le début de la séquence de démarrage d'un véhicule (40).

13. Procédé selon la revendication 11 ou 12, **caractérisé en ce qu'**à l'étape a) un nombre de personnes présentes dans la zone de champ d'image intérieure (34) est déterminé, de préférence un signal étant émis et/ou la mesure d'alcool n'étant pas démarrée en cas de détermination de plus d'une personne dans la zone de champ d'image intérieure (34).

14. Procédé selon la revendication 11, **caractérisé en ce qu'**à l'étape b) l'émetteur de signaux (20) envoie un signal infrarouge qui est de préférence alternativement activé et désactivé, ou au moins un signal lumineux qui comprend de préférence alternativement une lumière rouge et une lumière jaune et/ou l'intensité lumineuse d'un signal lumineux est variable.

15. Procédé selon la revendication 11, **caractérisé en ce qu'**à partir du début de la mesure d'alcool l'unité de prise de vues (30) enregistre des modifications à la limite entre la zone de champ d'image intérieure (34) et la zone de champ d'image extérieure (36), et l'unité d'analyse et de commande (54) analyse ces modifications.
